# EUROPEAN PATENT APPLICATION

(11) **EP 2 447 247 A1**
(43) Date of publication of application: **02.05.2012**
(21) Application number: 10013633.2
(22) Date of filing: 14.10.2010
(51) Int. Cl.: C07C 213/00

(54) **Process for the synthesis of chiral propargylic alcohols**

(71) Applicant: Lonza Ltd, 4052 Basel (CH)
(72) Inventor: The designation of the inventor has not yet been filed

(57) **Abstract**

A process for the synthesis of chiral propargylic alcohols.

## Description

### Description

The invention is directed to a process for the preparation of chiral propargylic alcohols, which are key intermediates for the preparation of pharmaceuticals and agrochemicals and as precursors for compounds in the materials sciences.

Jiang et al. disclosed in Tetrahedron Lett. 2002, 43, 8323-8325 and J. Org. Chem. 2002, 67, 9449-9451 the reaction of acetylene derivatives with aldehydes and ketones in the presence of equimolar amounts of a Zn(II) compound to give several racemic propargylic alcohols. Chiral compounds are not mentioned at all.

WO-A-95/20389, WO-A-96/37457, WO 98/30543 and WO 98/30540 disclose several processes for the production of chiral propargylic alcohols useful for the synthesis of pharmaceuticals. WO-A-98/51676 disclose a process wherein by addition of a first chiral and optionally a second additive in a zinc(II) mediated reaction the chiral product is obtained in high enantiomeric excess. The disadvantage of said process is the use of high amounts of expensive zinc catalysts and chiral compounds.

A main task for the present invention was therefore to supply an alternative process for the production of chiral propargylic alcohol with high enantiomeric excess. A further problem was to reduce the amounts of catalyst and other components to be added during the reaction in order to facilitate the workup procedures of the product and to promote industrial production.

The problem is solved by the process of claim 1. The inventive process comprises the addition of a starting amount of the chiral product to the reaction as a chiral mediator, which allows to reduce the amount of further chiral auxiliaries. Presence of the chiral product from the beginning of the reaction has the advantageous side effect that the amount of the zinc(II) catalyst can be reduced compared to processes known in the art. Furthermore, the addition of the compound of formula I allows to dispense with chiral auxiliaries, while still the chiral product is formed in high enantiomeric excess (*ee*).

Claimed is a process for the preparation of chiral compounds of the formula 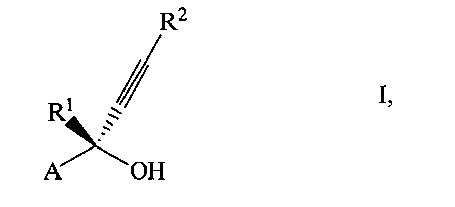 or mirror image, wherein
R¹ is selected from the group consisting of hydrogen, C₁₋₆-alkyl and (C₁₋₆-alkoxy)carbonyl, any alkyl or alkoxy optionally being substituted with one or more halogen atoms,
R² is selected from the group consisting of aryl, aralkyl, C₁₋₆-alkyl and (1'-R³)-C₃₋₆-cycloalkyl wherein R³ is hydrogen, methyl or ethyl, and wherein any aryl, aralkyl, alkyl is optionally substituted with one or more halogen atoms, and

A is selected from the group consisting of C₁₋₂₀-alkyl, C₃₋₆-cycloalkyl, aryl and aralkyl, any cycloalkyl, aryl and aralkyl optionally being annullated to one or more further 5 to 7 membered carbocyclic or heterocyclic rings, and wherein any alkyl, cycloalkyl, aryl and aralkyl is optionally substituted with one or more substituents selected from halogen atoms, cyano,
C₁₋₆-alkyl, C₃₋₆-cycloalkyl, -NR⁴R⁵, -SR⁶ and/or -OR⁷, with R⁶ is C₁₋₆-alkyl, optionally substituted with one or more halogen atoms,
R⁷ is hydrogen or C₁₋₆-alkyl, optionally substituted with one or more halogen atoms, or, where
(a) R⁴ and R⁵ are independently selected from hydrogen or C₁₋₆-alkyl, or
(b) R⁴ is hydrogen and R⁵ is C₂₋₇-acyl or (C₁₋₆-alkoxy)carbonyl, wherein each acyl and alkoxy in R⁵ in turn is optionally substituted with one or more halogen atoms, or
(c) R⁴ and R⁵ together with the nitrogen atom form a 5 to 7 membered heterocyclic ring, or
(d) R⁴ and R⁵ together are =CH-aryl, the aryl moiety optionally being substituted with one or more substituents selected from halogen atoms, -NH₂, -NH(C₁₋₆-alkyl), -N(C₁₋₆-alkyl)₂ or C₁₋₆-alkyl, or
(e) R⁴ and R⁵ together are =CH-N(C₁₋₆-alkyl)₂,
R⁶ is C₁₋₆-alkyl, optionally substituted with one or more halogen atoms, and
R⁷ is hydrogen or C₁₋₆-alkyl, optionally substituted with one or more halogen atoms, or, wherein A and R¹ together form a 5 to 7 membered carbocyclic or heterocyclic rings, optionally substituted with one or more substituents selected from halogen atoms, cyano, C₁₋₆-alkyl, C₃₋₆-cycloalkyl, -NR⁴R⁵, -SR⁶ and/or -OR⁷, wherein R³, R⁴, R⁵, R⁶ and R⁷ are as defined above, and wherein each alkyl and cycloalkyl substituent attached to A in turn is optionally substituted with one or more halogen atom, said process comprising the steps of
(i) reacting a protic chiral auxiliary with a diorganylzinc(II) compound, in the presence of an aprotic solvent, at a temperature in the range of 0 to 40 °C, and
(ii) keeping the mixture of step (i), preferably under stirring, in a first maturation period until the reaction is completed, but of at least 20 min, and
(iii) adding to the mixture obtained after step (ii) a compound of formula 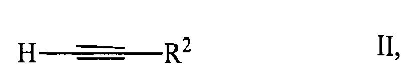 wherein R² is as defined above,
(iv) keeping the mixture of step (iii), preferably under stirring, in a second maturation period until the reaction is completed, but of at least 10 min, and
(v) adding a compound of formula 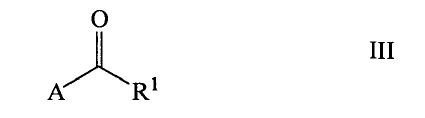 wherein A and R¹ are as defined above, and an organolithium base at a temperature in the range of 0 to 40 °C, and
(vi) keeping the mixture obtained in step (v) to 10 to 50 °C until the reaction is completed, to obtain the compound of formula I.

The major advantages of the present process are the reduction of the zinc(II) catalyst in view of the compound of formula III, the need of only one protic compound to first react with the zinc(II) catalyst, especially the possibility to avoid addition of fluorinated alcohols,

In contrast to known processes which requires the addition of two different proton sources, wherein an additional proton source can be methanol, ethanol, propanol, isopropyl alcohol, butanol, isobutanol, *sec*-butanol, *tert*-butanol, pentanol, (CH₃)₃CCH₂OH, (CH₃)₃CCH(CH₃)OH, Cl₃CCH₂OH, CF₃CH₂OH, CH₂=CHCH₂OH, (CH₃)₂NCH₂CH₂OH or even another chiral compound. The present process can be carried out with only one proton source, which at the same time acts as a chiral auxiliary. A preferred proton source in that sense is an ephedrine derivative, more preferably a phenylnorephedrine (PNE).

Here and hereinbelow the term "alkyl" represents a linear or branched alkyl group. By using the form "C₁₋ₙ-alkyl" the alkyl group is meant having 1 to n carbon atoms. C₁₋₆-alkyl represents for example methyl, ethyl, propyl, isopropyl, butyl, isobutyl, *sec*-butyl, *tert*-butyl, pentyl and hexyl.

Here and hereinbelow the term "dialkyl" independently means to alkyl groups attached to a connecting atom. For example in a dialkylzinc (II) compound, two alkyl groups are attached to zinc, whereas in dialkylamino the two alkyl groups are attached to nitrogen.

Here and hereinbelow the term "alkoxy" represents a linear or branched alkoxy group. By using the form "C₁₋ₙ-alkoxy" the alkyl group is meant having 1 to n carbon atoms. C₁₋₆-alkoxy represents for example methoxy, ethoxy, propoxy, isopropoxy, butoxy, isobutoxy, *sec*-butoxy, *tert*-butoxy, pentyloxy and hexyloxy.

Here and hereinbelow the term "cycloalkyl" represents a cycloaliphatic group having 3 carbon atoms or more. Cycloalkyl represents mono- and polycyclic ring systems such as cyclopentyl, cyclohexyl, cycloheptyl, cyclooctyl, adamantyl or norbornyl.

Here and hereinbelow the term "aryl" represents an aromatic group, preferably phenyl or naphthyl.

Here and hereinbelow the term "aralkyl" represents a group having 7 or more carbon atoms, consisting of an alkyl and an aryl moiety, wherein the alkyl moiety of the aralkyl residue is a C₁₋₈ alkyl group and the aryl moiety is selected from the group consisting of phenyl, naphthyl, furanyl, thienyl, benzo[b]furanyl, benzo[b]thienyl.

The present process relies on a specific order of addition of the compounds of the diorganylzinc(II) compound, the compounds of formulae II and III comprising the two maturation periods of steps (ii) and (iv), respectively. The term "until the reaction is completed" in steps (ii), (iv) and (vi) means that at least 90% conversion, preferably at least 95%, more preferably 98%, is reached in the respective step. The course of conversion can be followed for example by calorimetric measurements, "React IR" or FT-IR. Also possible are off-line methods such as gas chromatography or HPLC. It is possible to establish a correlation between conversion and the output of analytical methods easily with computer aided systems. We suggest that in the first maturation period a first catalytic species if formed, while in the second maturation step a second catalytic species is formed. The first catalytic species might comprise a compound of formula (alkyl)Zn(chiral auxiliary) which might be solved in the mixture or aggregated. The second catalytic species might comprise a compound of formula (C≡C-R²)Zn(chiral auxiliary), wherein R² is as defined above. By using diethylzinc ethane evolution of approx. 1 equivalent in respect to diethylzinc could be observed in steps (ii) and (iv), respectively. Ethane formation could be detected during the diethylzinc addition. The ethane release was observed with a delay with respect to the diethylzinc addition. It is assumed that ethane was first dissolved in the reaction solution and then released to the gas phase. ¹H-NMR analysis shows that some ethane remained dissolved in the reaction mixture. The structures of the catalytic species can be only proposed because of the difficulties to separate the catalytic species from the respective precursors. Especially, since catalytic species would be highly sensitive to air and humidity.

In step (v) the addition of the compound of formula III and the organolithium base are fed simultaneously, either separately or as a mixture. Advantageously, dosage of the organolithium base starts ahead of the dosage of the compound of formula III, preferably up to 20 min ahead, more preferably up to about 10 min ahead.

The process is designed to obtain the compound of formula I with an enantiomeric purity (*ep*) of at least 90%, preferably with an *ep* of at least 95%, more preferred of at least 96%, and even more preferred of at least 97%.

The protic chiral auxiliary induces the formation of the desired enantiomer during reaction of the compounds of formulae II and III. The expression "protic chiral auxiliary" means that the chiral auxiliary comprises at least one abstractable proton, most preferred in a hydroxyl group.

In a preferred embodiment the chiral auxiliary is selected from protic *N,N*-disubstituted ephedrine derivatives.

Suitable protic *N,N*-disubstituted ephedrine derivatives are for example diastereoisomers of 2-(di-C₁₋₄-alkylamino)-1-phenyl-propan-1-ols such as 2-(dimethylamino)-1-phenyl-propan-1-ol, 2-(diethylamino)-1-phenyl-propan-1-ol, 2-(diisopropylamino)-1-phenyl-propan-1-ol, and 2-(dibutylamino)-1-phenyl-propan-1-ol; 2-(*N,N*-C₄₋₆-alkylene)-1-phenyl-propan-1-ols, such as 1-phenyl-2-(piperidinyl)propan-1-ol and 1-phenyl-2-(pyrrolidinyl)propan-1-ol, and 2-(1-hetaryl)-1-phenyl-propan-1-ols, such as 1-phenyl-2-(1-pyridinyl)propan-1-ol, 1-phenyl-2-(1-piridinyl)propan-1-ol and. More specific examples are (1*R*,2*S*)-2-(dimethylamino)-1-phenyl-propan-1-ol (CAS [552-79-4]), (1*S*,2*R*)-2-(dimethylamino)-1-phenyl-propan-1-ol (CAS [42151-56-4]), (1*R*,2*R*)-2-(dimethylamino)-1-phenyl-propan-1-ol (CAS [14222-20-9]), (1*S*,2*S*)-2-(dimethylamino)-1-phenyl-propan-1-ol (CAS [51018-28-1]), (1*R*,2*S*)-1-phenyl-2-(pyrrolidinyl)propan-1-ol (CAS [127641-25-2]), (1*S*,2*R*)-1-phenyl-2-(pyrrolidinyl)propan-1-ol (CAS [123620-80-4] = (1*S*,2*R*)-PNE), (1*R*,2*R*)-1-phenyl-2-(pyrrolidinyl)propan-1-ol and (1*S*,2*S*)-1-phenyl-2-(pyrrolidinyl)propan-1-ol.

In a preferred embodiment the protic chiral auxiliary is (1*R*,2*S*)-phenylnorephedrine ((1*R*,2*S*)-PNE or (1*S*,2*R*)-1-phenyl-2-(pyrrolidinyl)propan-1-ol) to obtain ((*S*)-2-(2-amino-5-chlorophenyl)-4-cyclopropyl-1,1,1-trifluorobut-3-yn-2-ol (DMP-266) or one of its salts, from 1-(2-amino-5-chlorophenyl)-2,2,2-trifluoroethanone and cyclopropylacetylene.

The amount of the zinc(II) catalyst needed in the reaction can be reduced remarkably compared to processes known in the art. It must be noted that the amount of the zinc(II) catalyst can be much lower than the amount of the chiral auxiliary.

In a preferred embodiment the molar ratio of the protic chiral auxiliary to the diorganylzinc(II) compound is in the range of 1.5:1 1 to 1:1, preferably in the range of 1.3:1 1 to 1.2:1, most preferred at about 1.24:1.

The chiral auxiliary mediates the catalytic process. Although one would expect that zinc(II) catalyst and the protic chiral auxiliary form a zinc(II) complex with a certain stoichiometry it is not necessary to add the chiral auxiliary and the zinc(II) catalyst in equimolar amounts. Preferably the amount of the chiral auxiliary is slightly higher than the amount of the diorganylzinc(II) catalyst.

Suitable diorganylzinc(II) compounds are for example selected from di(C₁₋₈-alkyl) and di(C₃₋₆-cycloalkyl), wherein the alkyl moieties are selected from the group consisting of methyl, ethyl, propyl, isopropyl, butyl, isobutyl, *sec*-butyl and *tert*-butyl, pentyl, hexyl, heptyl, and octyl, and wherein the cycloalkyl moieties are selected from the group consisting of cyclopropyl, cyclobutyl, cyclopentyl and cyclohexyl.

In another embodiment the diorganylzinc(II) compound is diphenylzinc or Zn(OTf)₂, wherein OTf denotes a "triflate" (trifluoromethanesulfonate) group.

In a preferred embodiment in step (i) the molar ratio of the protic chiral auxiliary to the compound of formula III is in the range of 1:1 to 1:10, preferably in the range of 1:2 to 1:6, more preferably of 1:3 to 1:6.

Addition of the compound of formula III can be carried out at a temperature from 0 to +40 °C, preferably from +10 to about +30 °C.

In a preferred embodiment the compound of formula II is selected from the group consisting of terminal C₃₋₈-alkylalkynes, cyclopropylacetylene, (1'-methyl)-cyclopropylacetylene and phenylacetylene.

It is recommended, that in step (iii) the compound of formula II is used in a molar ratio to the compound of formula III of 1:0.6 to 1:1.3

In a preferred embodiment the compounds of formula II are selected from the group consisting of *p*-methylbenzaldehyde, *p*-fluorobenzaldehyde, *p*-cyanobenzaldehyde, *p*-methoxybenzaldehyde, naphthalenealdehyde, cinnamaldehyde, C₃₋₂₀-alkane aldehydes, cycloheane carbaldehyde, cyclohexyl metyl ketone, methyl 4-metylcyclohexyl ketone, 1,1,1-trifluoroacetophenone and 2-(trifluoroaceto)-4-chloro-anilin.

In a further preferred embodiment the organolithium base is added in a molar ratio to the compound of formula III in the range of 1:0.8 to 1:1.5, preferably of 1:0.8 to 1:1.2.

A suitable organolithium base in the present process is selected from the group consisting of (C₁₋₆-alkyl)lithium, lithium diisopropylamide (LDA), lithium hexamethyldisilazide (LiHMDS), phenyllithium, and naphthyllithium.

Preferably the organolithium base is an organolithium compound or a lithium organic salt.

In preferred embodiment such organometallic lithium compound is selected from the group consisting of phenyllithium and (C₁₋₆-alkyl)lithium.

Preferably said (C₁₋₆-alkyl)lithium is selected from the group consisting of methyllithium, *n*-butyllithium, *sec*-butyllithium, *tert*-butyllithium, and hexyllithium.

In a further preferred embodiment the lithium organic salt is a lithium C₁₋₆-alkoxide.

During the addition of the base the reaction mixture is preferably kept at a temperature from about + 10 to +30 °C.

In the present process the aprotic solvent preferably is selected from the group consisting of aprotic non-polar solvents, aprotic polar solvents and mixtures thereof.

The solvents of agents added in solution may be selected independently of each other. Particularly preferred the solvent is selected from the group consisting of tetrahydrofuran, benzene, chlorobenzene, *o-, m-, p*-dichlorobenzene, dichloromethane, toluene, *o*-, *m*-, and *p*-xylene, hexanes, heptanes, cyclohexane, pentane, 1,4-dioxane, cyclohexane, diethyl ether, *tert-*butyl methyl ether, diisopropyl ether, *N*-methylpyrrolidine, and mixtures thereof.

### Examples:

The chiral alkynylation reaction (Examples 1, 2, and 4) was performed two times with the respective starting compounds. Once using (1*R*,2*S*)-pyrrolidinyl-norephedrine ((1*R*,2*S*)-PNE) as ligand and once using (1*R*,2*S*)-pyrrolidinyl-norephedrine ((1*S*,2*R*)-PNE) as ligand. This allowed the unambiguous assignment of the two enantiomers of the products by HPLC. In the experimental details below, only the experiments using (1*S*,2*R*)-PNE are described in detail because there was no major difference between (1*R*,2*S*)-PNE and (1*S*,2*R*)-PNE. For the all examples using cyclopropylacetylene addition to a diethylzinc catalyst, an ethane release could be observed as well as described in Example 1. The configuration of the products of example 2 to 4 were tentatively assigned based on the assumption that reaction in presence of ligand (1*S*,2*R*)-PNE gives preferably the product with (*R*)-configuration in analogy to Example 1 (SD573 process), where the configuration of both enantiomers are well known. In the SD573 process (1*R*,2*S*)-PNE gives preferably the product with (*S*)-configuration. Procedures for analytical methods A to D are attached after the examples.

In all cyclisation examples, except where not expressively mentioned, the *ee* was not measured since in all cyclisation examples with final *ee*-measurement the product (for example DMP-266) always corresponded to the *ee* of the respective starting compound, for example in case of cyclisation of SD573-MSA or SD573 free base (CAS [209412-27-7], 99.6% *ee*) to DMP-266.

### Example 1: 6: (S)-2-(2-Amino-5-chlorophenyl)-4-cyclopropyl-1,1,1-trifluorobut-3-yn-2-ol mesylate (2:3 mol/mol) (SD573-MSA)

A solution of (1*R*,2*S*)-PNE (18.1%-w/w, 171.6 g, 151 mmol) in a THF/toluene mixture (9:1-w/w) was charged in a vessel and cooled to 17 °C. A solution of diethylzinc in toluene (29%-w/w, 52.0 g, 122 mmol) was added at 15 to 20 °C and the mixture was aged at said temperature for 30 min. Ethane (approx. 1 equivalent in respect to diethylzinc) was formed during the diethylzinc addition and partially released from the reaction mixture. The ethane release is observed with a delay with respect to the diethylzinc addition, since ethane is first dissolved in the reaction solution and then released to the gas phase. According to ¹H-NMR analysis some ethane remained dissolved in the reaction mixture. A solution of cyclopropylacetylene in toluene (70%-w/w, 57.0 g, 600 mmol) was added at 15 to 20 °C and the resulting mixture was aged at 20 °C for 1 h. During the addition of cyclopropylacetylene additional ethane (approx. 1 equivalent in respect to diethylzinc) was formed and released to the gas phase. A solution of butyllithium (BuLi) in toluene (157.6 g, 2.92 mol/kg, 460 mmol) and a solution of 1-(2-amino-5-chlorophenyl)-2,2,2-trifluoroethanone (SD570) (40.1%-w/w, 278.0 g, 500 mmol) in THF/toluene (1:1-w/w) were added in parallel to the reaction mixture at 20 °C within 180 min. The addition of BuLi was started 10 min in advance of the SD570 addition. Butane was formed during BuLi addition. However, most of the butane remained dissolved in the reaction mixture and only weak gas formation was observed. The course of reaction can be followed online, for example by calorimetric measurements or by "React IR" also called "In-Situ FTIR". After complete addition of SD570 the reaction mixture was stirred for 30 min at 20 °C, then heated to 30 °C over a period of 60 min and aged for 6 h at 30 °C. The reaction mixture was stirred at 0 °C overnight, diluted with toluene (218 g) at 20 °C and quenched by addition of aqueous citric acid (1 M, 375 g). After stirring for 15 min the phases were separated and the aqueous phase was discarded. The organic phase successively was washed with water (76 g), aqueous NaHCO₃ solution (5%-w/w, 200 g), and again water (100 g). The organic phase was partially concentrated, then diluted with toluene (250 g), again partially concentrated and diluted with toluene (976 g residue). The enantiomeric purity *(ep)* of (*S*)-2-(2-amino-5-chlorophenyl)-4-cyclopropyl-1,1,1-trifluorobut-3-yn-2-ol (SD573) in the crude product was approx. 96 to 97% according to Method B. Although not belonging to the preparation process, described is also a process to transfer the product in a more stable form as a methanesulfonic acid salt. The residue was diluted with isopropyl alcohol (126.6 g). Thenn methanesulfonic acid (43.3 g) was added over a period of 30 min at 30 °C. Seeding crystals (between 1 and 10 mg) were added and the mixture aged for 30 min at 30 °C. A second portion of methanesulfonic acid (26.5 g) was added over a period of 60 min at 30 °C. The resulting solution was aged for 30 min at 30 °C and later cooled to 5 °C over a period of 60 min. After further aging at 5 °C for 30 min, the product was filtered and washed with cold toluene/isopropyl alcohol (10:1-w/w, 262 g) at 5 °C. The wet methanesulfonic acid salt of SD573 ((*S*)-2-(2-Amino-5-chlorophenyl)-4-cyclopropyl-1,1,1-trifluorobut-3-yn-2-ol mesylate (2:3 mol/mol, SD573-MSA) was dried *in vacuo* at 40 °C to obtain 188.3 g (432 mmol, 86.5% yield). SD573-MSA was obtained with a purity of 99.9% and 99.7% ep, according to Method A.

### Example 2: 7: (R)-2-(4-Aminobiphenyl-3-yl)-4-cyclopropyl-1,1,1-trifluorobut-3-yn-2-ol methanesulfonate (1:1 mol/mol)

(1*S*,2*R*)-PNE (20.3 g, 18.0 mmol) in THF/toluene (9:1-w/w, 18.2%-w/w) was charged under nitrogen to a dry, jacketed 150 mL-reactor with agitator. Diethylzinc in toluene (29.9%-w/w, 6.48 g, 15.7 mmol) was added by syringe keeping the temperature at 17 to 22 °C and the mixture was aged for 30 min at 17 °C. Cyclopropylacetylene in toluene (69.6%-w/w, 6.84 g, 72.0 mmol) was added at 17 °C and the resulting mixture was aged for about 60 min at 20 °C. To the reaction mixture BuLi in toluene (3.06 mol/kg, 19.9 g, 60.9 mmol) and (1-(4-aminobiphenyl-3-yl)-2,2,2-trifluoroethanone (CN46225) (43.0%-w/w, 37.0 g, 60 mmol) in THF/toluene (1:1-w/w) were added in parallel over a period of 3 h at 20 °C. The addition of BuLi was started about 10 min in advance of the CN46225 addition. After complete addition of BuLi and CN46225, the reaction mixture was stirred for 30 min at 20 °C, then heated over a period of 1 h to 30 °C and aged for 6 h at 30 °C. The reaction mixture was stirred overnight at 0 °C. HPLC indicated 94.3 area% conversion and 95.6% *ep*, according to Method B. The reaction mixture was diluted with toluene (27.6 g) at room temperature and quenched by adding aqueous citric acid (1 M, 45.3 g). The phases were separated and the aqueous phase was discarded. The organic phase successively was washed with water (9.1 g), aqueous NaHCO₃ (5%-w/w, 24.2 g) and water (12.0 g). The organic phase was heated under reduced pressure to partly remove THF while toluene is added to finally reach a THF poor residue (54.5 g) of (*R*)-2-(4-aminobiphenyl-3-yl)-4-cyclopropyl-1,1,1-trifluorobut-3-yn-2-ol (CN46630). The residue was diluted with isopropyl alcohol (16.7 g) and toluene (60.0 g). A first portion of methanesulfonic (5.48 g, 57.0 mmol) was added by a syringe pump over a period of 30 min at 30 °C. Seeding crystals (a small portion between 1 and 10 mg) were added and the mixture was aged for 30 min at 30 °C. A second portion of methanesulfonic (2.88 g, 30.0 mmol) was added by syringe pump over a period of 45 min at 30 °C. The mixture was stepwise aged and cooled over 1 h 45 min to finally reach 5 °C. The product was filtered, and the filter cake was washed with toluene/isopropyl alcohol (10:1-w/w, 27.0 g) and dried *in vacuo* at 40 °C. The dry product (R)-2-(4-aminobiphenyl-3-yl)-4-cyclopropyl-1,1,1-trifluorobut-3-yn-2-ol methanesulfonate (1:1 mol/mol, CN46630-MSA) (15.2 g, 35.6 mmol, 59% yield) was obtained as an off-white solid (99.4% purity and 99.7% *ep,* according to Method B). The combined mother liquor and wash liquor was concentrated (46.7 g residue). During storage overnight at 3 °C a white solid crystallized from the residue. The product was filtered, washed with toluene, before toluene/isopropyl alcohol (10:1-w/w, 10 g) was added. After stirring for 60 min at 30 °C the mixture was cooled to 3 °C and filtered. The product was washed with toluene/isopropyl alcohol (10:1-w/w) and dried *in vacuo* at 40 °C. CN46630-MSA (second crop, 3.8 g, 8.0 mmol, 13% yield) was obtained as off-white solid (89.7% purity at 99.7% *ep*, according to Method B).

### Example 3: (R)-4-(Cyclopropylethynyl)-6-phenyl-4-(trifluoromethyl)-1,4-dihydro-2H-3,1-benzoxazin-2-one

(*R*)-2-(4-Aminobiphenyl-3-yl)-4-cyclopropyl-1,1,1-trifluorobut-3-yn-2-ol methanesulfonate (CN46630-MSA) (14.7 g, 34.4 mmol) in ethyl acetate/heptanes (1:1 v/v, 27.9 g) was charged in a jacketed 150 mL-reactor with agitator and off-gas scrubber with caustic soda. After addition of aqueous Na₂CO₃ (12%-w/w, 32.3 g, 36.4 mmol, formation of gas during addition!) the mixture was stirred for 15 min at 15 °C. The aqueous phase was separated and discarded. Aqueous Na₂CO₃ (12%-w/w, 41 g, 46 mol) and ethyl acetate (20 g) were charged to the organic phase. Triphosgene (4.41 g, 14.9 mmol) was added in portions over a period of 25 min at 10 °C. The reaction mixture was stirred for 2 h at 8 °C. The mixture was diluted with ethyl acetate (45 g) and the phases were separated. The aqueous phase was discarded. The organic phase was washed with water (12 mL), dried over MgSO₄, concentrated and dried at 50 °C under reduced pressure to obtain (*R*)-4-(Cyclopropylethynyl)-6-phenyl-4-(trifluoromethyl)-1,4-dihydro-2*H*-3,1-benzoxazin-2-one (CN46685) (12.1 g, 33.9 mmol, 98%) as a yellowish solid (99.5% purity, according to Method C).

### Example 4: (R)-2-(2-Amino-5-fluorophenyl)-4-cyclopropyl-1,1,1-trifluorobut-3-yn-2-ol methanesulfonate (2:3 mol/mol)

(1*S*,2*R*)-PNE (18.2%-w/w, 20.3 g, 18.0 mmol) in THF/toluene (9:1-w/w) was charged under nitrogen to a dry, jacketed 150 mL-reactor with agitator. Diethylzinc in toluene (29.9%-w/w, 6.20 g, 15.0 mmol) was added by syringe while keeping the temperature at 17 to 22 °C. Then the mixture was aged for 30 min at 17 °C. Cyclopropylacetylene in toluene (69.6%-w/w, 6.82 g, 71.8 mmol) was added at 17 °C and the reaction mixture was aged for 60 min at 20 °C. To the reaction mixture BuLi in toluene (19.3 g, 3.06 mol/kg, 59.1 mmol) and 1-(2-amino-5-fluorophenyl)-2,2,2-trifluoroethanone (CAS [214288-07-0], CN46221) (36.9%-w/w, 33.7 g, 60.0 mmol) in THF/toluene (1:1-w/w) were added in parallel over a period of 3 h at 20 °C. The addition of BuLi was kept 10 min in advance of the CN46221 addition. After completed addition the reaction mixture was stirred at 20 °C for 30 min, heated over a period of 60 min to 30 °C and aged for 6 h at 30 °C. The reaction mixture was stirred overnight at 0 °C. HPLC indicated 82.4% conversion and 96.0% *ep* of (*R*)-2-(2-amino-5-fluorophenyl)-4-cyclopropyl-1,1,1-trifluorobut-3-yn-2-ol (CN46619) according to Method B. The reaction mixture was diluted with toluene (27.6 g) and quenched by adding aqueous citric acid (1 M, 45.3 g). The phases were separated and the aqueous phase was discarded. The organic phase successively was washed with water (9.1 g), aqueous NaHCO₃ (5%-w/w, 24.2 g) and water (12.0 g). The organic phase was alternating concentrated and diluted with toluene to remove THF. The obtained residue (51.0 g) was diluted with isopropyl alcohol (16.7 g) and toluene (60.0 g). Methanesulfonic (8.36 g, 87.0 mmol) was added by a syringe pump over a period of 75 min at 30 °C. The mixture was aged and cooled stepwise over 2 h 10 min to reach 5 °C before the mixture was filtered. The filter cake was washed with toluene/isopropyl alcohol (10:1-w/w, 27.0 g) and dried under reduced pressure at 40 °C. The dry product (*R*)-2-(2-amino-5-fluorophenyl)-4-cyclopropyl-1,1,1-trifluorobut-3-yn-2-ol methanesulfonate (2:3 mol/mol, CN46619-MSA) (19.46 g, 46.6 mmol, 78% yield) was obtained as a yellowish solid (99.8%-w/w by ¹H-NMR, and 99.8% *ep*, according to Method B).

### Example 5: (R)-4-(Cyclopropylethynyl)-6-fluoro-4-(trifluoromethyl)-1,4-dihydro-2H-3,1-benzoxazin-2-one

(*R*)-2-(2-Amino-5-fluorophenyl)-4-cyclopropyl-1,1,1-trifluorobut-3-yn-2-ol methanesulfonate (CN46619-MSA) (14.0 g, 33.5 mmol) in ethyl acetate/heptanes (40 g, 6/4 v/v) was charged to a jacketed 150 mL-reactor with agitator and off-gas scrubber with caustic soda. After addition of aqueous Na₂CO₃ (12%-w/w, 26.9 g, 30.3 mmol) the mixture was stirred for 5 min at 15 °C. The aqueous phase was separated and discarded. Aqueous Na₂CO₃ (12%-w/w, 34.1 g, 38.4 mmol) was charged to the organic phase, then triphosgene (3.73 g, 12.6 mmol) was added in portions over a period of 25 min at 10 °C. The reaction mixture was stirred for 2 h at 8 °C. The mixture was charged with heptanes (15.9 g), the phases were separated and the aqueous phase discarded. The organic phase was washed with water (12 mL), dried over MgSO₄ and concentrated to dryness. After drying under vacuum at 50 °C, the product (*R*)-4-(cyclopropylethynyl)-6-fluoro-4-(trifluoromethyl)-1,4-dihydro-2*H*-3,1-benzoxazin-2-one (CN46686) (9.78 g, 32.7 mmol, 97%) was obtained as a yellowish solid (99.4% purity, according to Method C).

### Example 6: Cyclisation of SD573 with diphosgene

Aqueous Na₂CO₃ (12%-w/w, 183 g, 0.206 mol) was charged to SD573-MSA (SD573.MSA = (*S*)-2-(2'-amino-5'-chlorophenyl)-4-cyclopropyl-1,1,1-trifluorobut-3-yn-2-ol mesylate (2:3 mol/mol) = methanesulfonate of the S-enantiomer of the compound of formula II, wherein R¹ is trifluoromethyl, R² is cyclopropylethynyl R³ is Cl in *para*-position in respect of the amino group, R⁴ and R⁵ are hydrogen; 100 g, 0.23 mol, corresponding to 66.8 g of SD573 free base, 99.6% *ee*) in ethyl acetate/heptanes (203 g, 1.5:1 v/v). The mixture was stirred for 5 min at 15 °C. Hydrolysis of the mesylate ended at about pH 9.0 of the aqueous phase. Then a phase separation was performed and the aqueous phase was removed. Aqueous Na₂CO₃ (12%-w/w, 232 g, 0.262 mol) was charged to the organic phase. To the biphasic mixture, liquid diphosgene (24 g, 120 mmol) was added in 90 min at 12 °C. After a conversion of more than 99.7% was reached, according to Method C, heptanes (204 g) were charged. The reaction mixture was then heated to 20 °C, the aqueous phase was separated and discarded, while the organic phase was washed with water (about 80 g). The organic layer was heated under reduced pressure, ethyl acetate distilled off and heptanes were charged to the reaction mixture to achieve a residual ethyl acetate content of 5.5%-w/w and a ratio of heptanes to organic matter of 10 L/kg in view of originally added SD573-MSA. Then the mixture was heated to dissolve all organic matter. The solution was seeded with 0.8 g of DMP-266 (DMP-266 = *S*-enantiomer of the compound of formula I, wherein R¹ is trifluoromethyl, R² is cyclopropylethynyl R³ is Cl in *para*-position in respect of the amino group, R⁴ and R⁵ are hydrogen) at 55 °C and stirred for about 15 min at 55 °C. The mixture was then cooled to 50 °C and hold for 120 min. Then the mixture was further cooled within 2 h from 50 °C to 25 °C and within another 2 h ramp to about -10 °C. Finally, the mixture was stirred for about 1 h at -10 °C maximum and then filtered. The filter cake (wet product) was washed with pre-cooled heptanes (2×50 mL) at 0 °C maximum. The solid was dried *in vacuo* to yield 94.2% (68.4 g, 216 mmol) of DMP-266 at a purity of 99.9%-w/w according to Method D. The sample comprises 99.8%-w/w *S*-enantiomer, i.e. an enantiomeric excess (*ee*) of 99.6%.

### Example 7: Cyclisation of SD573 with diphosgene

Aqueous Na₂CO₃ (12%-w/w 91.5 g, 0.103 mol) was charged to SD573-MSA (50 g, 0.115 mol, corresponding to 33.4 g of SD573 free base, 99.6% *ee*) in ethyl acetate/heptanes (101.5 g, 1.5/1 v/v). The mixture was stirred for 5 min at 15 °C. Hydrolysis of the mesylate ended at pH 6.4 in the aqueous phase. A phase separation was performed and the aqueous phase was removed. The remaining organic phase was cooled to 12 °C and aqueous Na₂CO₃ (12%-w/w, 106 g, 0.12 mol) was charged. To the biphasic mixture, liquid diphosgene (11.4 g, 57 mmol) was added in 90 min at 12 °C. After a total conversion was reached according to Method C, heptanes (68.8 g) was charged. The reaction mixture was then heated to 20 °C, stirred 30 min and the aqueous phase was removed. The organic phase was heated under reduced pressure, ethyl acetate was distilled off and heptanes charged to the reaction mixture to achieve a residual ethyl acetate content of 4.4 w% and a ratio of heptanes to organic matter of 6.5 L/kg in view of originally added SD573-MSA. Then the obtained mixture was heated to dissolve all organic matter. The solution was seeded with DMP-266 (0.4 g) at 55 °C and stirred for about 15 min at 55 °C. The mixture was then cooled to 50 °C and hold for 120 min. The mixture was further cooled in 2 h from 50°C to 25°C and within another 2 h to -10 °C maximum. The mixture finally was stirred for about 1 h at -13 °C and then filtered. The filter cake (wet product) was washed with pre-cooled heptanes (2×50 mL) at 0 °C maximum. The solid was dried *in vacuo* to yield 92.1% (33.45 g, 105 mmol) of DMP-266 of a purity of 99.9%-w/w according to Method D. The sample comprises 99.8%-w/w *S* enantiomer, i.e. 99.6% *ee.*

### Example 8: Cyclisation of SD573 with triphosgene

SD573-MSA (50 g, 0.114 mol, corresponding to 33.4 g of SD573 free base, 99.6% ee) was dissolved in an ethyl acetate/heptanes mixture (164 g, 1:1 v/v) and charged with aqueous Na₂CO₃ (12%-w/w, 91.5 g, 0.104 mol). After hydrolysis of the mesylate a pH about 7.0 was measured in the aqueous phase. The mixture was stirred for at least 5 min at 15 °C. Then a phase separation was performed and the aqueous phase was removed. The mixture was cooled below 12 °C and aqueous Na₂CO₃ (12%-w/w, 116 g, 0.131 mol) was charged. To the biphasic mixture, triphosgene (12.5 g, 42 mmol) was added at 10 °C maximum in five portions within 90 min. The mixture was stirred further 15 min below 15 °C. After a total conversion was reached according to Method C, heptanes (54 g) was charged. The reaction mixture was then heated to 20 °C and the aqueous phase was removed. The organic phase was washed with water (40 g) and then heated under reduced pressure, ethyl acetate distilled off and heptanes charged to the reaction mixture to achieve a residual ethyl acetate content of 4.6%-w/w and a ratio of heptanes to organic matter of 6.5 L/kg in view of originally added SD573-MSA. The solution was seeded with DMP 266 (0.4 g) at 57 °C and stepwise cooled under stirring stirred at -10 °C within 2 h 15 min. The mixture was stirred at -10 °C maximum overnight and then filtered. The filter cake was washed with pre-cooled heptanes (2×25 mL) at 0 °C maximum. The solid was dried in vacuo to yield 95% of DMP-266 (34.22 g, 108 mmol) at a purity of 100%-w/w, according to Method D. The sample comprises 99.8%-w/w S-enantiomer, i.e. 99.6% *ee*.

### Example 9: Cyclisation of SD573 with triphosgene

SD573-MSA (100 g, 0.23 mol, corresponding to 66.8 g of SD573 free base, 99.6% *ee*) was dissolved in ethyl acetate/heptanes (203 g, 1.5:1 v/v) and charged with aqueous Na₂CO₃ (12%w/w, 183 g, 0.207 mol) at about 15 °C. A pH of 7 to 9 was reached in the aqueous phase. The mixture was stirred for 5 min at 15 °C. Then the phase separation was performed and the aqueous phase was removed. The mixture was cooled below 12 °C and aqueous Na₂CO₃ (12%-w/w, 232 g, 0.263 mol) was charged. To the biphasic mixture, triphosgene (24.08 g, 81 mmol) was added in 10 portions within 120 min at less than 12 °C. The mixture was stirred further 10 min at about 12°C. After a total conversion was reached according to Method C, heptanes (204 g) were charged. The reaction mixture was then heated to 20 °C and the aqueous phase was removed. The organic phase was washed with water (80 g) and then heated under reduced pressure to partially remove ethyl acetate, while heptanes were charged to the reaction mixture to achieve a residual ethyl acetate content of 5.8%-w/w and a ratio of heptanes to organic matter of 7.0 L/kg in view of originally added SD573-MSA. The solution was seeded with DMP-266 (0.8 g) at 55 °C and stirred for 15 min. Then the mixture was cooled to 50 °C within 20 min, hold for 2 h, cooled to 25 °C within 2 h, cooled to about -10 °C within 2 h. After cooling to about -10 °C and stirring overnight the mixture was filtered. The isolated product was washed with pre-cooled heptanes (2×50 mL) at -10 °C maximum. The solid was dried *in vacuo* to yield 85.4% DMP-266 (62 g, 19.6 mmol) at a purity of 100%-w/w, according to Method D. The sample comprises 99.8%-w/w S-enantiomer, i.e. 99.6% *ee*.

### Example 10: Cyclisation of SD573 with triphosgene

Aqueous Na₂CO₃ (14%-w/w, 135 g, 0.178 mol) was charged to SD573 free base (33.4 g, 0.115 mol) in ethyl acetate/heptanes (70.4 g of 45:55 v/v) at 15 °C. The mixture was cooled to 8 °C and triphosgene in heptanes (26.8%-w/w, 112 g, 101 mmol) was added within 60 min, while the temperature was kept at 5 °C to 12 °C. After 60 min a total conversion was reached, according to Method C. The reaction mixture was heated to 25 °C. Then a phase separation was performed and the aqueous phase was removed. The organic phase was heated under reduced pressure, ethyl acetate partly was distilled off and heptanes were charged to the reaction mixture to achieve a residual ethyl acetate content of about 2.5%-w/w and a ratio of heptanes to organic matter of about 15 L/kg in view of originally added SD573 free base. Then the mixture was heated to dissolve all organic matter and afterwards seeded with DMP-266 (overall 1.4 g) at 55 °C. No product crystallized and therefore the organic phase was heated under reduced pressure to partially remove ethyl acetate, while heptanes were charged to the reaction mixture to achieve a residual ethyl acetate content of less then 3% (w/w) and a ratio of heptanes to organic matter of about 15 L/kg in view of originally added SD573 free base. Then the mixture was heated to dissolve all organic matter and afterwards seeded with DMP-266 (1.5 g) at 51 °C and stirred for about 140 h at 51 °C. The slurry was stepwise cooled under stirring within 4 h to reach -15 °C. The slurry was stirred for 16 h at -15 °C and then filtered. The isolated product was washed with pre-cooled heptanes (2×55 mL) at -10 °C maximum. The solid was dried *in vacuo* to yield 92.9% (33.7 g, 107 mmol) of DMP-266, at a purity of 99.6%-w/w, according to Method D. The sample comprises 99.8%-w/w *S*-enantiomer, i.e. 99.6% *ee*.

### Example 11: Cyclisation of SD573 with triphosgene

Aqueous Na₂CO₃ (12%-w/w, 91.5 g, 0.103 mol) was charged to SD573-MSA (50 g, 0.115 mol, corresponding to 33.4 g of SD573 free base) in ethyl acetate/heptanes (90.8 g, 55/45 v/v). The mixture was stirred for 5 min at 15 °C resulting in a pH 6.8 of the aqueous phase. Then a phase separation was performed and the aqueous phase was removed. The organic phase was heated under reduced pressure and the solvent was partially removed (32.3 g, 41 mL) to obtain a ratio of SD573 free base to solvent of about 1:1.75 (w/w). The distillate contained about 53.2 w% of ethyl acetate. The mixture comprising the SD573 free base was cooled to 12 °C and aqueous Na₂CO₃ (12%-w/w, 96 g, 0.109 mol) was charged. To the biphasic mixture, triphosgene in ethyl acetate (31%-w/w, 32.7 g, 34 mmol) was added in 66 min at 7 to 12 °C. The mixture was stirred 15 min at 12 °C maximum. After a conversion of 90.2% was reached, according to Method C, additional heptanes (86 g) were charged and the reaction mixture was heated to 20 °C. Then a phase separation was performed and the aqueous phase was removed. The organic phase was heated under reduced pressure to partially remove ethyl acetate while heptanes were charged to the organic phase to achieve a residual ethyl acetate content of 6.8%-w/w (target 3 to 7%-w/w) and a ratio of heptanes to organic matter of 6.8 L/kg in view of originally added SD573-MSA. The solution was seeded with DMP-266 (0.4 g) at 47 °C and stirred for 150 min at 47 to 55 °C. Then the mixture was slowly cooled to -10 °C and filtered. The filter cake was washed with pre-cooled heptanes (2×25 mL) at -10 °C maximum. The solid was dried *in vacuo* to yield 81.1% (29.46 g, 0.093 mmol) of DMP-266 of a purity of 97.2%-w/w according to Method D.

### Example 12: Cyclisation of SD573 with triphosgene

Aqueous Na₂CO₃ (12%-w/w, 275.1 g, 0.311 mol) was charged to SD573-MSA (150 g, 0.345 mol, corresponding to 100.2 g of SD573 free base) in ethyl acetate/heptanes (272.1 g, 55/45 v/v). After stirring the mixture for 5 min at 15 °C a pH of 7.7 was measured. Then a phase separation was performed and the aqueous phase was discarded. The organic phase (ethyl acetate/heptanes ratio of 61.5/38.5 w/w) was split into 3 parts each comprising about 33 g of SD573 free base. With an aim to test the stability of SD573 free base in ethyl acetate/heptanes mixtures, the 1^{st} part was stored for 4 days at 4 °C before performing Example 12.1, the 2^{nd} part was stored for 7 days at 4 °C before performing Example 12.2, and the 3^{rd} part was stored for 10 days at 4 °C before performing Example 12.3.

### Example 12.1:

The 1^{st} part of the organic phase of Example 12 (123.5 g) was heated under reduced pressure to partially remove the solvent until the distillate contained 60 w% of ethyl acetate (about 33 g). The remaining mixture was cooled to 12 °C and aqueous Na₂CO₃ (12%-w/w, 117 g, 0.132 mol) was charged. To the biphasic mixture, triphosgene in ethyl acetate (36%-w/w, 35 g, 42 mmol) was added in 60 min at less than 12 °C. The mixture was stirred 15 min at less than 12 °C. After a total conversion was reached according to Method C, heptanes (86 g) were charged and the reaction mixture was heated to 20 °C. Then a phase separation was performed and the aqueous phase was removed. The organic phase was washed with water (40 g). The organic phase was heated under reduced pressure to party remove ethyl acetate, while heptanes were charged to the reaction mixture to achieve a residual ethyl acetate content of 5.5%-w/w (target 3 to 7%-w/w). A ratio of heptanes to organic matter of 6.3 L/kg in view of originally added SD573-MSA was obtained for crystallisation. The solution was seeded with DMP-266 (0.4 g) at 57 °C and stirred for 15 min at the seeding temperature. The mixture was stepwise cooled under stirring to -15 °C within 6 h 20 min, stirred overnight at -10 °C and finally filtered. The filter cake was washed with pre-cooled heptanes (2×25 mL) at -10 °C maximum. The solid was dried *in vacuo* to yield 89.4% (32.17 g, 102 mmol) of DMP-266 at a purity of 100%-w/w according to Method D.

### Example 12.2:

The 2^{nd} part of the organic phase of Example 12 (122.0 g) was heated under reduced pressure to partially remove the solvent until the distillate contained 53 w% of ethyl acetate (about 31 g). The mixture was cooled to 12 °C and aqueous Na₂CO₃ (12%-w/w, 117 g, 0.132 mol) was charged. To the biphasic mixture triphosgene in ethyl acetate (36%-w/w, 35 g, 42 mmol) was added in 60 min at 12 °C maximum. The mixture was stirred for 15 min at 12 °C maximum. A total conversion was obtained according to Method C. Heptanes (86 g) were charged and the reaction mixture was heated to 20 °C. Then a phase separation was performed and the aqueous phase was removed. The organic phase was washed with water (40 g) and then heated under reduced pressure to partially remove ethyl acetate, while heptanes were charged to the reaction mixture to achieve a residual ethyl acetate content of 5.7%-w/w (target 3 to 7%-w/w). A ratio of heptanes to organic matter of 6.4 L/kg in view of originally added SD573-MSA was obtained for crystallisation. The mixture was seeded with DMP-266 (0.4 g) at 57 °C and stepwise cooled under stirring within 6 h to reach -15 °C. Then the mixture was stirred at -10 °C overnight and filtered. The filter cake was washed with pre-cooled heptanes (2×50 mL) at -10 °C maximum. The solid was dried *in vacuo* to yield 89.5% (32.21 g, 102 mmol) of DMP-266 at a purity of 100%-w/w according to Method D.

### Example 12.3:

The 3^{rd} part of the organic phase of Example 12 (122.5 g) was heated under reduced pressure to partially remove the solvent until the distillate contained 53.6 w% of ethyl acetate (about 32.3 g). The mixture was cooled to 9 °C before aqueous Na₂CO₃ (12%-w/w, 117 g, 0.132 mol) was charged. To the biphasic mixture triphosgene in ethyl acetate (36%-w/w, 35 g, 42 mmol) was added in 60 min at 12 °C maximum and the mixture stirred for 1 h at 12 °C maximum. A total conversion was obtained according to Method C. Heptanes (86 g) were charged and the reaction mixture was heated to 20 °C. A phase separation was performed and the aqueous phase was removed. The organic phase was washed with water (40 g) and then heated under reduced pressure to partially remove ethyl acetate, while heptanes were charged to the reaction mixture to achieve a residual ethyl acetate content of 5.8%-w/w. A ratio of heptanes to organic matter of 6.2 L/kg in view of originally added SD573-MSA was obtained for crystallisation. The mixture was seeded with DMP-266 (0.4 g) at 57 °C and stepwise cooled under stirring to -15 °C within 6 h. The mixture was stirred at -10 °C overnight and then filtered. The filter cake was washed with pre-cooled heptanes (2×25 mL) at -10 °C maximum. The solid was dried *in vacuo* to yield 90% of DMP-266 (32.4 g, 103 mmol) at a purity of 100%-w/w according to Method D.

### Example 13: Cyclisation of SD573 with triphosgene

Aqueous Na₂CO₃ (14%-w/w, 160 g, 0.211 mol) was charged to SD573-MSA (100 g, 0.229 mol, corresponding to 66.8 g of SD573 free base) in ethyl acetate/heptanes (158.8 g 1/1 v/v). The mixture was stirred at approx. 15 °C resulting in a pH 6.8 of the aqueous phase. Then a phase separation was performed and the aqueous phase was removed. The organic phase was cooled to 12 °C and aqueous Na₂CO₃ (14%-w/w, 214 g, 0.283 mol) was charged. To the biphasic mixture triphosgene in ethyl acetate (35.7%-w/w, 67.2 g, 81 mmol) was added in 60 min at 12 °C maximum. The mixture was stirred for 30 min at 12°C maximum. Heptanes (96 g) were charged and a total conversion was obtained according to Method C. The reaction mixture was heated to 20 °C. Then a phase separation was performed and the aqueous phase was removed. Aqueous Na₂CO₃ (14%-w/w, 92 g, 0.121 mol) was added to the organic phase and stirred for 25 min at 20 °C. Then a phase separation was performed and the aqueous phase was removed. The organic phase (360 g) was split into two parts.

### Example 13.1:

The 1^{st} part of the organic phase of Example 8 (180 g) was washed with water (80 g), phase separation was performed and the aqueous phase was removed. Then the organic phase was heated under reduced pressure, ethyl acetate partially distilled off and heptanes charged to the reaction mixture to achieve a residual ethyl acetate content of 3%-w/w (target 3 to 7%-w/w) was obtained. Finally total 10 L/kg SD573-MSA heptanes was achieved for the crystallisation. The solution was seeded with DMP-266 (0.2 g) at 55 °C and stepwise cooled under stirring to -15 °C within 7 h. The mixture was stirred at -15 °C overnight and then filtered. The filter cake was washed with pre-cooled heptanes (2×50 mL) at -10 °C maximum. The solid was dried *in vacuo* to yield 93% (33.47 g, 105 mmol) of DMP-266, the purity is 98.8%-w/w, according to Method D.

### Example 13.2:

The 2^{nd} part of the organic phase of Example 8 (180 g) was heated under reduced pressure to partially remove ethyl acetate, while heptanes were charged to the reaction mixture, to achieve a residual ethyl acetate content of 3.4%-w/w (target 3 to 7%-w/w). A ratio of heptanes to organic matter of 10 L/kg in view of originally added SD573-MSA was obtained for crystallisation. The mixture was seeded with 0.2 g of DMP-266 at 55 °C and stepwise cooled under stirring within 6 h 40 min to reach -15 °C. The mixture was stirred at -10 °C overnight and then filtered. The filter cake was washed with pre-cooled heptanes (2×50 mL) at -10 °C maximum. The solid was dried *in vacuo* to yield 96% (34.87 g, 110 mmol) of DMP-266 at a purity of 97.7%-w/w according to Method D.

### Example 14: Cyclisation of SD573 with triphosgene

Aqueous Na₂CO₃ (14%-w/w 80 g, 0.106 mol) was charged to SD573-MSA (50 g, 0.115 mol, corresponding to 33.4 g of SD573 free base) in of ethyl acetate/heptanes (79.4 g, 1/1 v/v) and charged. After stirring for 15 min a pH of 6.4 was measured in the aqueous phase. The mixture was stirred for 5 min at 15 °C. Then a phase separation was performed and the aqueous phase was removed. The mixture was cooled to 12 °C and aqueous Na₂CO₃ (14%-w/w, 107 g, 0.141 mol) was charged. Triphosgene in ethyl acetate (35.7%-w/w, 33.6 g, 40.5 mmol) was added to the biphasic mixture for 60 min at 12 °C maximum. The mixture was stirred 30 min at 12°C maximum. Heptanes (48 g) were charged and a total conversion was obtained according Method C. The reaction mixture was heated to 20 °C. Then a phase separation was performed and the aqueous phase was removed. The organic phase was washed with water (80 g) and then heated under reduced pressure, to partially remove ethyl acetate, while heptanes were charged to the reaction mixture, to achieve a residual ethyl acetate content of 3.2%-w/w. A ratio of heptanes to organic matter of 9.6 L/kg in view of originally added SD573-MSA was obtained for crystallisation. The mixture was seeded with 0.2 g of DMP-266 at 55 °C, and stepwise cooled under stirring within 7 h to reach -15 °C. The mixture was stirred at -15 °C overnight and then filtered. The filter cake was washed with pre-cooled heptanes (50 mL) at -10 °C maximum. The solid was dried *in vacuo* to yield 97% (34.49 g, 110 mmol) of DMP-266 at a purity of 96.5%w/w according to Method D.

### Example 15: Cyclisation of SD573 with triphosgene

Aqueous Na₂CO₃ (14%-w/w, 80 g, 0.106 mol) was charged to SD573-MSA (50 g, 0.114 mol, corresponding to 33.4 g of SD573 free base) in ethyl acetate/heptanes (79.4 g, 1/1 v/v). After stirring for 15 min a pH of 6.1 was measured in the aqueous phase. The mixture was stirred for 5 min at 15 °C. Then the phase separation was performed and the aqueous phase was removed. The mixture was cooled to 12 °C and aqueous Na₂CO₃ (14% w/w, 135 g, 0.178 mol) was charged. To the biphasic mixture triphosgene in ethyl acetate (35.7%-w/w, 33.6 g, 40.5 mmol) was added in 60 min at 12 °C maximum. The mixture was stirred 30 min at 12°C maximum. Heptanes (48 g) were charged and a total conversion was obtained according to Method C. The reaction mixture was heated to 20 °C. Then a phase separation was performed and the aqueous phase was removed. The organic phase was washed with water (80 g) and then heated under reduced pressure to partially remove ethyl acetate, while heptanes were charged to the reaction mixture, to achieve a residual ethyl acetate content of 2.8%-w/w. A ratio of heptanes to organic matter of 9.5 L/kg in view of originally added SD573-MSA was obtained for crystallisation. The solution was seeded with DMP-266 (0.2 g) at 55 °C and stepwise cooled under stirring within 4 h 35 min to reach to -15 °C. The mixture was stirred overnight at -15 °C and then filtered. The filter cake was washed with pre-cooled heptanes (2×50 mL) at -10 °C maximum. The solid was dried *in vacuo* to yield 97.6% of DMP-266 (35.12 g, 111 mmol) at a purity of 95.1%-w/w according to Method D.

### Example 16: Cyclisation of SD573 with phosgene

Aqueous Na₂CO₃ (12%-w/w, 183 g, 0.207 mol) was charged to SD573-MSA (100 g, 0.228 mol, corresponding to 66.8 g of SD573 free base) in ethyl acetate/heptanes (203 g, 1.5:1 v/v). After stirring for 15 min a pH of 7.2 was measured in the aqueous phase. The mixture was stirred for 5 min at 15 °C. Then the phase separation was performed and the aqueous phase was removed. The mixture was cooled to 12 °C and aqueous Na₂CO₃ (12%-w/w, 232 g, 0.263 mol) was charged. Phosgene (24.8 g, 251 mmol) was added to the biphasic mixture in 90 min at 12 °C maximum. Heptanes (136 g) were charged to the mixture and a total conversion was obtained according to Method C. The reaction mixture was heated to 20 °C. Then a phase separation was performed and the aqueous phase was removed. The organic phase was washed with water (80 g) and then heated under reduced pressure to partially remove ethyl acetate, while heptanes were charged to the reaction mixture, to achieve a residual ethyl acetate content of less then 7%-w/w. A ratio of heptanes to organic matter of 9.7 L/kg in view of originally added SD573-MSA was obtained for crystallisation. The solution was seeded with DMP-266 (0.8 g) at 55 °C, stepwise cooled under stirring within 6 h 15 min to reach -15 °C and then filtered. The filter cake was washed with pre-cooled heptanes (2×50 mL) at 0 °C maximum. The solid was dried *in vacuo* to yield 95.7% of DMP-266 (68.91 g, 218 mmol) at a purity of 100%-w/w according to Method D.

### Example 17: Cyclisation of SD573 with phosgene

SD573-MSA (50 g, 0.114 mol, corresponding to 33.4 g of SD573 free base) was dissolved in ethyl acetate/heptanes (102 g, 55:45 v/v) and charged with aqueous Na₂CO₃ (12%-w/w, 91 g, 0.103 mol). After stirring for 15 min a pH of about 7 was measured in the aqueous phase. The mixture was stirred for 5 min at 15 °C. Then the phase separation was performed and the aqueous phase was separated and discarded. The organic phase was cooled to 12 °C and charged with aqueous Na₂CO₃ (12%-w/w, 157 g, 0.178 mol). To the biphasic mixture phosgene (16.9 g, 171 mmol) was added in 130 min at 12 °C maximum. Heptanes (43 g) were charged and a total conversion was obtained according to Method C. The reaction mixture was heated to 20 °C. Then a phase separation was performed and the aqueous phase was removed. The organic phase was washed with water (80 g) and then heated under reduced pressure to partially remove ethyl acetate, while heptanes were charged to the reaction mixture, to achieve a residual ethyl acetate content of 3.5%-w/w. A ratio of heptanes to organic matter of ca. 10 L/kg in view of originally added SD573-MSA was obtained for crystallisation. The solution was seeded with DMP-266 (0.4 g) at 62 °C and stepwise cooled under stirring to -5 °C overnight and then filtered. The filter cake was washed with pre-cooled heptanes (2×50 mL) at 0 °C maximum. The solid was dried in *vacuo* to yield 93% of DMP-266 (33.86 g, 107 mmol) at a purity of 98.5%-w/w according to Method D.

### Comparative Example 1: Cyclisation of SD573 with triphosgene, homogeneous

Aqueous Na₂CO₃ (10.6 g, 0.126 mol) was charged at 25 °C to SD573 free base (25.13 g, 0.087 mol) in acetonitrile (25 mL) in a 500 mL reactor. The mixture was cooled to -12 °C and a solution of triphosgene in acetonitrile (19.7%-w/w, 63.63 g, 42 mmol) was added within 40 min at -10 to -5 °C. After 90 min a total conversion was reached according to Method C. The reaction mixture was heated to 25 °C, neutralized at 20 °C to 25 °C with Na₂CO₃, washed with water and then filtered through a G3 frit. The mixture was cooled to -10 °C and water (7.5 g) was added dropwise. The slurry was filtered through a G3 frit and the product was isolated. The wet cake was dried *in vacuo* to give the final product with 5% yield (1.89 g, 6 mmol). The purity was 97.3%-w/w according to Method D.

### Comparative Example 2: Cyclisation of SD573 with triphosgene, homogenous

To SD573 free base (25.04 g, 0.086 mol) dissolved in acetone (25 mL) in a 500 mL reactor, Na₂CO₃ (10.6 g, 0.126 mol) and water (50 mL) were charged at 25 °C. The mixture was cooled to -12 °C and a solution of triphosgene in acetonitrile (24%-w/w, 52 g, 42 mmol) was added at -10 to -5 °C within 55 min. After 60 min a conversion of 98.1%-w/w was reached, according to Method C. The reaction mixture was heated to 25 °C. After further 100 min a conversion of final 98.8%-w/w was reached, according to Method C. Triphosgene (0.69 g) was added. After 180 min a total conversion was reached, according to Method C. The reaction mixture was neutralized at 20 °C to 25 °C with Na₂CO₃ and then filtered through a G3 frit. The filter was washed with water (12.5 g). To the filtrate water (100 mL) was added at 25 °C. Because after 15 h no product precipitated, the mixture was cooled to -10 °C and filtered to obtain crop 1. To the filtrate water (200 mL) was added at -10 °C and the suspension was filtered again to obtain crops 2. Precipitation was repeated with further water (100 mL) addition to the filtrate of crop 2 to obtain crop 3. The combined crops (1 to 3) of wet product were dried in vacuo to obtain 84.5% yield (22.39 g, 71 mmol). The purity was 96.9%-w/w according to Method D.

### Comparative Example 3: Cyclisation of SD573 with triphosgene, homogenous

To SD573 free base (25.11 g, 0.087 mol) dissolved in THF (25 mL) in a 500 mL reactor, Na₂CO₃ (10.6 g, 0.126 mol) and water (50 mL) was charged at 25 °C. The mixture was cooled to -12 °C and a solution of triphosgene in THF (22.1%-w/w, 56.5 g, 42 mmol) was added between -10 °C to -5 °C within 36 min. After 120 min a conversion of 96.2%-w/w was reached, according to Method C. The reaction mixture was heated to 25 °C. After further 100 min a conversion of 97.7% (w/w) was reached, according to Method C. Triphosgene (0.68 g) was added. Further small portions of triphosgene were added until 99.6% (w/w) conversion was reached. The reaction mixture was neutralized between 20 to 25 °C with Na₂CO₃ and then filtered through a G3 frit. To the mixture water (325 g) was added at 25 °C. The mixture was cooled to 0 °C and filtered through a G3 frit (crop 1). To the product remaining in the vessel further water (200 mL) was added at 5 °C; and the mixture was filtered (crop 2). To the product remaining in the vessel further water (100 mL) was added at 5 °C; and the mixture was filtered (crop 3). The combined crops (1 to 3) of wet product were dried *in vacuo* to obtain 56.5% yield (15.53 g, 49 mmol). The purity was 98.1%-w/w according to Method D.

### Analytical Methods:

**Method A**: (HPLC method used for the determination of the enantiomeric purity)
Column: Chiralpak^{®} AD, 250×4.6 mm; Temperature: 40°C; Flow: 1.0 mL/min; Mobile Phase: hexane/isopropyl alcohol = 75:25 (v/v); UV Detection: 260 nm

**Method B**: (HPLC method used for conversion, purity and enantiomeric purity):
Column: Chiralpak^{®} AD-H, 250×4.6 mm; Temperature: 40 °C; Flow: 1.0 mL/min; Mobile phase: hexane/isopropyl alcohol = 89:11 (v/v); UV Detection: 260 nm

**Method C:** (HPLC method used for the determination of the purity):
Column: Zorbax^{®} RX-C18, 250×4.6 mm, 5 micrometer; Temperature: 40°C; Flow: 1.5 mL/min; Mobile phase A: 50% buffer / 50% MeCN; Mobile phase B: MeCN; Buffer: 0.1% H₃PO₄ in water, pH adjusted to 3.6; Gradient: 0 min 0% B to 30 min 90% B; UV Detection: 250 nm

**Method D**: (HPLC method used for the determination of the purity):
Column: Zorbax^{®} SB-CN, 150x4.6 mm; Temperature: 40°C; Flow: 1.5 mL/min; Mobile phase A: 90% water/10% MeOH + 0.05% TFA (v/v); Mobile phase B: 90% water/10% MeOH + 0.05% TFA (v/v); Gradient: 16 min 40% to 50% B, 7 min to 65% B, 5 min to 70% B, 1 min to 80% of B, 2 min hold 80% B, 1 min to 40% B; UV Detection: 250 nm

*ee* = enantiomeric excess = ((*S*)-(*R*))/((*S*)+(*R*))

*ep* = enantiomeric purity = (*S*)/((*S*)+(*R*))

## Claims

1. A process for the preparation of a compound of formula 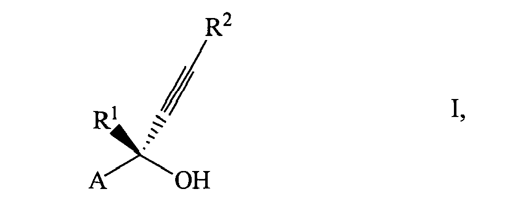 or mirror image, wherein
R¹ is selected from the group consisting of hydrogen, C₁₋₆-alkyl and (C₁₋₆-alkoxy)carbonyl, any alkyl or alkoxy optionally being substituted with one or more halogen atoms,
R² is selected from the group consisting of aryl, aralkyl, C₁₋₆-alkyl and (1'-R³)-C₃₋₆-cycloalkyl wherein R³ is hydrogen, methyl or ethyl, and wherein any aryl, aralkyl, alkyl is optionally substituted with one or more halogen atoms, and
A is selected from the group consisting of C₁₋₂₀-alkyl, C₃₋₆-cycloalkyl, aryl and aralkyl, any cycloalkyl, aryl and aralkyl optionally being annullated to one or more further 5 to 7 membered carbocyclic or heterocyclic rings, and wherein any alkyl, cycloalkyl, aryl and aralkyl is optionally substituted with one or more substituents selected from halogen atoms, cyano, C₁₋₆-alkyl, C₃₋₆-cycloalkyl, -NR⁴R⁵, -SR⁶ and/or -OR⁷, with R⁶ is C₁₋₆-alkyl, optionally substituted with one or more halogen atoms,
R⁷ is hydrogen or C₁₋₆-alkyl, optionally substituted with one or more halogen atoms, or, where
(a) R⁴ and R⁵ are independently selected from hydrogen or C₁₋₆-alkyl, or
(b) R⁴ is hydrogen and R⁵ is C₂₋₇-acyl or (C₁₋₆-alkoxy)carbonyl, wherein each acyl and alkoxy in R⁵ in turn is optionally substituted with one or more halogen atoms, or
(c) R⁴ and R⁵ together with the nitrogen atom form a 5 to 7 membered heterocyclic ring, or
(d) R⁴ and R⁵ together are =CH-aryl, the aryl moiety optionally being substituted with one or more substituents selected from halogen atoms, -NH₂, -NH(C₁₋₆-alkyl), -N(C₁₋₆-alkyl)₂ or C₁₋₆-alkyl, or
(e) R⁴ and R⁵ together are =CH-N(C₁₋₆-alkyl)₂,
R⁶ is C₁₋₆-alkyl, optionally substituted with one or more halogen atoms, and
R⁷ is hydrogen or C₁₋₆-alkyl, optionally substituted with one or more halogen atoms, or, wherein A and R¹ together form a 5 to 7 membered carbocyclic or heterocyclic rings, optionally substituted with one or more substituents selected from halogen atoms, cyano, C₁₋₆-alkyl, C₃₋₆-cycloalkyl, -NR⁴R⁵, -SR⁶ and/or -OR⁷, wherein R³, R⁴, R⁵, R⁶ and R⁷ are as defined above, and wherein each alkyl and cycloalkyl substituent attached to A in turn is optionally substituted with one or more halogen atom,
said process comprising the steps of
(i) reacting a protic chiral auxiliary with a diorganylzinc(II) compound, in the presence of an aprotic solvent, at a temperature in the range of 0 to 40 °C, and
(ii) keeping the mixture of step (i), preferably under stirring, in a first maturation period until the reaction is completed, but of at least 20 min, and
(iii) adding to the mixture obtained after step (ii) a compound of formula 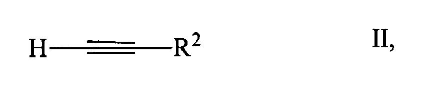 wherein R² is as defined above, and
(iv) keeping the mixture of step (iii), preferably under stirring, in a second maturation period until the reaction is completed, but of at least 10 min, and
(v) adding to the mixture obtained after step (iv) a compound of formula 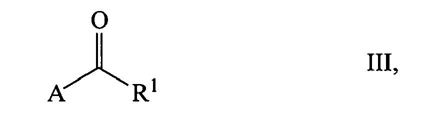 wherein A and R¹ are as defined above, and an organolithium base at a temperature in the range of 0 to 40 °C, and
(vi) keeping the mixture obtained in step (v) to 10 to 50 °C until the reaction is completed, to obtain the compound of formula I.

2. The process of claim 1, wherein the protic chiral auxiliary is selected from the group consisting of *N,N*-disubstituted ephedrine derivatives.

3. The process of claims 1 or 2, wherein the molar ratio of the protic chiral auxiliary to the diorganylzinc(II) compound is in the range of 1.5:1 to 1:1.

4. The process of any of claims 1 to 3, wherein the diorganylzinc(II) compound is selected from the group consisting of di(C₁₋₈-alkyl) and di(C₃₋₆-cycloalkyl), wherein the alkyl moieties are selected from the group consisting of methyl, ethyl, propyl, isopropyl, butyl, isobutyl, *sec*-butyl and *tert*-butyl, pentyl, hexyl, heptyl, and octyl, and wherein the cycloalkyl moieties are selected from the group consisting of cyclopropyl, cyclobutyl, cyclopentyl and cyclohexyl.

5. The process of any of claims 1 to 4, wherein in step (i) the molar ratio of the protic chiral auxiliary to the compound of formula III is in the range of 1:1 to 1:10, preferably in the range of 1:2 to 1:6, more preferably of 1:3 to 1:6.

6. The process of any of claims 1 to 5, wherein in step (iii) the compound of formula II is used in a molar ratio to the compound of formula III of 1:0.6 to 1:1.3.

7. The process of any of claims 1 to 6, wherein the organolithium base is added in a molar ratio to the compound of formula III from 1:0.8 to 1:1.5.

8. The process of any of claims 1 to 9, wherein the organolithium base is selected from the group consisting of (C₁₋₆-alkyl)lithium, lithium diisopropylamide (LDA), lithium hexamethyldisilazide (LiHMDS), phenyllithium, and naphthyllithium.

9. The process of claim 8, wherein the (C₁₋₆-alkyl)lithium is selected from the group consisting of methyllithium, *n*-butyllithium, *sec*-butyllithium, *tert*-butyllithium, and hexyllithium.

10. The process of any of claims 1 to 8, wherein the temperature during the addition of the base is of from +10 to +30 °C.

11. The process of any of claims 1 to 10, wherein the aprotic solvent is selected from the group consisting of aprotic non-polar solvents, aprotic polar solvents and mixtures thereof.
